# EUROPEAN PATENT SPECIFICATION

(11) **EP 1 425 026 B1**
(45) Date of publication and mention of the grant of the patent: **05.05.2010**
(21) Application number: 02748536.6
(22) Date of filing: 16.08.2002
(51) Int. Cl.: A61K 36/84, A61P 25/20

(54) **VALERIANA OFFICINALIS EXTRACT FOR TREATING SLEEP-INDUCED APNEA OR HYPOPNEA**
VALERIANA OFFICINALIS EXTRAKT ZUR BEHANDLUNG VON SCHLAFAPNOE ODER HYPOPNOE
VALERIANA OFFICINALIS POUR TRAITER L' APNÉE DU SOMMEIL OU HYPOPNÉE

(30) Priority: 14.09.2001 US 318865 P
(43) Date of publication of application: 09.06.2004
(73) Proprietor: Max-Zeller Söhne AG, 8590 Romanshorn (CH)
(72) Inventor: BRATTSTRÖM, Axel, 78476 Allensbach (DE)
(74) Representative: Ritscher, Thomas
(86) International application number: PCT/CH2002/000448
(87) International publication number: WO 2003/024464

(56) References cited:
- DE-A- 19 614 847
- DE-A- 19 702 168
- US-B1- 6 399 116
- PATENT ABSTRACTS OF JAPAN vol. 005, no. 079 (C-056), 23 May 1981 (1981-05-23) & JP 56 029521 A (SHOWA DENKO KK;OTHERS: 01), 24 March 1981 (1981-03-24)
- SANER B ET AL: "THERAPIE DER SCHLAFSTOERUNGEN IM ALTER" DEUTSCHE MEDIZINISCHE WOCHENSCHRIFT, GEORG THIEME, STUTTGART,, DE, vol. 122, no. 51/52, December 1997 (1997-12), pages 1599-1604, XP000944706 ISSN: 0012-0472

## Description

### Field of the Invention

This invention is concerned with the use of Valeriana officinalis extract in treating human patients suffering from sleep-induced apnea or hypopnea (also termed apnoea and hypopnoea, respectively). Patients suffering from hypopnea (too shallow breathing) or apnea (more or less periodical interruptions of breathing) while sleeping tend to develop sleep deficiencies when awake that may lead to reduced vigilance and increasing sleepiness.

### Prior Art

Many chemical substances (sleeping aids) are known that induce sleep, such as the barbiturates, the benzodiazepines as well as various sedatives from synthetic and phytopharmacological fields, notably including valerian extract but there is an undimished demand for improved sleeping aids.

As a result, somnology has become a field of intensive studies and "sleep quality" can be measured in a variety of ways. Measurement of periods of rapid eye movement (REM) and periods non-rapid eye movement (NREM) during sleep is an acknowledged method. Yet, the mechanisms that do regulate sleep at the cellular level are largely unknown and, specifically, no effective means for treating sleep-induced apnea and Hypopnea in humans, nor methods of treating these states in humans, have become known.

Certain substances, such as typically caffeine and theophylline as well as certain amphetamines are known as "wake-up drugs" are effective as stimulants of the central nervous system inducing vigilance and increasing the time spent awake. However, eventually sleep is a must and the "quality of sleep" seems to be responsible, at least to a substantial extent, for the degree of recovery. It appears that such substances are effective because they act as antagonists or agonists for adenosine receptors.

It is suspected that adenosine formation regulated by neuronal activity is at the core of transition to sleep, and research about antagonists to adenosine A₁ and A₂ receptors (Carley, D. W. et al: Sleep 20,12, (1997), p.1093-1098) has indicated the possibility of a dose-dependent suppression of apnea during NREM sleep in rats injected intraperitoneally with phenylisopropyl adenosine (an A₁ receptor agonist), 2-p-(2-carboxyethyl)-phenethylamino-5'-N-ethylcarboxamido-adenosme hydrochloride (an A₂ receptor agonist) and N-[(1 S,trans)-2-hydroxycyclopentyl]-adenosine (an A₁ receptor agonist). Frequently, administration of these substances in animal tests resulted in hypotension and hypothermia. Obviously, substances of this type would not be suitable for use as medicaments.

Now, while a general correlation between blood adenosine metabolism and sleep in humans has been established, the relation between such mechanisms and the quality of the ensuing sleep is not known to an extent sufficient to provide an effective treatment of hypopnea or apnea. More specifically, one would have expected that such treatment might be achieved with carefully controlled dosages of substances from the "wake-up drug" family rather than from the group of sleeping aids including sedatives.

### OBJECT AND BRIEF SUMMARY OF THE INVENTION

Accordingly, it is a general object of the present invention to provide for compositions that are effective in treating sleep-induced apnea or hypopnea in humans.

In the course of research leading to the present invention and based upon functional assays of various plant extracts it has been found surprisingly that valerian extract, a well known sedative (and a member of the group of substances believed to contribute to rather than alleviate hypopnea or apnea) is capable of actually reducing or even eliminating sleep-induced apnea and hypopnea in human patients.

Such research included various combinations of known phytochemical compositions, and it was found that constituents of Valeriana officinalis commonly contained in conventional valerian extracts might provide an effective treatment of sleep-induced apnea and hypopnea, both if used as such or in combination with other plant extracts, e.g. those containing constituents of Humulus lupulus and/or conventional adjuvants and additives of the type used in the Galenic art.

In PATENT ABSTRACTS OF JAPAN, vol. 005, no. 079 (C-056) & JP 56 029521) it is suggested that roots of a Valeriana species (Valeriana Fauriei) are effective against snoring. In that reference "snoring" is specified as "having not physical or clinical causes".

On the other hand, typical symptoms of sleep-induced apnea or hypopnea are (a) an interruption of respiratory air flow during at least 10 seconds, (b) a decrease of arterial oxygen saturation by at least 4 %, and (c) arousal (a measurable sleep interruption) causing daytime sleepiness and other negative physiological effects, such as hypertension (cf. "Prospective study of the association between sleep-disordered breathing and hypertension" , Paul E. Peppard et al, in "The New England Journal of Medicine", Vol. 342 (May 11, 2000), pp. 1378 - 1384).

### DEFINITIONS AND PREFERRED EMBODIMENTS OF THE INVENTION

The term "extract" as used herein relates to the product of extracting a plant material for a sufficient period of time (typically 1- 48 hours) with a suitable solvent, typically an organic solvent, carbon dioxide, and water, for mixtures of such advents with each other. Solvents frequently preferred are aqueous mixtures of lower alkanols (i.e. containing from 1 to 4 carbon atoms). Typical extraction temperatures are in the range of from about 0°C to about 100°C. Elevated pressures may be applied but extraction at ambient pressure is frequently preferred. Generally, suitable extracts may be liquid or dry and are generally obtained by removing the solvent from the primary product of extraction.

Extracts of Valeriana officinalis suitable for use according to the invention are obtainable commercially and are defined in various pharmacopoeias, e.g. the Swiss Pharmacopoeia Helvetica V, Monography 314, page 366. Such extracts will also be termed valerian extract herein for short. Valerian extracts for medical use and including use according to the invention should preferably be essentially free of valepotriates.

By the same token, extracts of various other plants including Humulus lupulus for medical use are defined pharmacologically and are obtainable commercially from various sources.

Extracts of Valeriana officinalis and of other medicinal plants mentioned herein for use according to the invention may be used in liquid or solid form as such, or in admixture with each other, as well as compounded with other additives and adjuvants used in the art of preparing medical preparations, e.g. diluents, binders, disintegrants, lubricants, anti-adherents, flow promotors. Typical examples of such additives include maltodextrine, microcrystalline cellulose, soja-derived polysaccharides, polyethylene glycol (e.g. Macrogol), magnesium stearate, colloidal anhydrous silica, propylene glycol, aroma and flavor components, such as vanilla aroma.

It is an advantage of the invention that safe dosages of the extracts mentioned herein have been well known for a long time. Typically, extract of Valeriana officinalis can be used safely at daily dosages of up to 1000 mg, and all such safe dosages are expected to be effective. Single administration before sleeping is preferred. Typical and preferred effective single dosages are in the range of from about 200 to about 500 mg/day. If valerian extract is used in combination with other extracts, the valerian component will preferably constitute at least about 50 % by weight of the extract ingredients of the composition, preferably at least about 75% by weight of the extract ingredients.

### BRIEF DESCRITPION OF THE DRAWINGS

The invention will be explained in more detail in the Examples which make reference to the drawings in which:
Figure 1 is a diagram of results of a binding study made with different extracts;
Figure 2 is another diagram showing the result of effects of various extracts upon receptor stimulation;
Figure 3 is a diagram showing relative inhibition of valerian extract compared with a known inhibitor; and
Figure 4 is a diagram similar to that of Fig. 3 for the relative inhibition of the lignane-type olivil derivative obtained from valerian extract termed "scholle-4".

### EXAMPLES

The following examples are given merely by way of illustration and to present experimental evidence for the effectiveness of the invention. However, no limitation to specific compositions, forms of dosage, or uses mentioned herein is intended.

### Example 1

Thirty patients (21 female aged 56 ± 10 years, 9 male aged 61 ± 3) with mild-moderate severity of sleep disorder were recruited out of 150 patients originally sent to the sleep laboratory for diagnostic reasons. In addition to standard anamnesis and clinical examinations the diagnosis was confirmed by polysomnography. Only those patients suffering from mild-moderate, non-organic insomnia were selected for this study.

Sleep disorders were reported to have occurred at least three times per week and for at least one month. Interfering neurological or medical clinical conditions as well as use of psychotropic substances or other medications known to interfere with sleep were excluded in the patient group of this study. Any other sleep disorder treatment had to be stopped at least two weeks preceding the study.

Patients were each examined twice by polysomnography at an interval of 14 days with one night of adaptation before recording. Patients went to bed at their usual sleep time. A first examination was used for objective confirmation of the diagnosis: non-organic insomnia. In the examinations, standard 17-channels were recorded (.5 EEG leads: C3-A2, C4-A1, FP1-A2, FP2-A1, C3-C4; 2 EOG: right and left; 2 EMG: submental and tibial; ECG, respiration: thoracic, abdominal, SA02, flow sensor; body position, and snoring microphone) to safely rule out any sleep-related disorders, periodic limb movement syndrome, or restless legs syndrome. Individual sleep stages were evaluated automatically using suitable software (Schwarzer Company, Comlab 32; version 2.8).

After such confirmation, treatment was started with two tablets each evening two hours before bedtime. Each tablet contained a fixed combination of 250 mg of standard dry valerian extract (Pharmacopoeia Helvetica V, Monography 314, page 366; product supplied by Zeller AG, Romanshorn, Switzerland, Batch No 011540) and 60 mg extract from Humulus lupulus (commercial product for medical use supplied by Zeller AG, Romanshom, Switzerland, Batch No 012450). A second polysomnographic examination two weeks after start of therapy was used for the response follow-up.

The results are summarized in Table 1 below. Values are given as mean values with their standard deviations. Variables from polysomnographic studies were compared pair-wise for biometric evaluation. The test is considered robust for a non-normal distribution if the number of cases is at least 30. Double-tailed tests were run. Patient compliance, determined from tablet counts, was good; i.e., all prescribed tablets were taken as directed. No patient exhibited or indicated adverse effects.

Evaluation of the results of the first examination by polysomnography indicated that nine patients of the group under study suffered from sleep-induced apnea of hypopnea. The results of the polysomnography tests at the start and after two weeks of the nine patients that suffered from sleep-induced apnea or hypopnea are tabulated in table 1 below.

**Tab. 1.**

| | Baseline | 2 Weeks |
|---|---|---|
| | n=9 | n=9 |
| Sleeping time (min) | 378.6 ± 42.8 | 372.7 ± 46.5 |
| Sleep efficacy (rating) | 78.2 ± 6.8 | 79.0 ± 4.9 |
| Apnea count | 31.8 ± 9.9 | 2.2 ± 1.0 |
| Hypopnea count | 30.3 ± 10.7 | 6.0 ± 2.6 |
| Apnea index (counts/hour) | 5.30 ± 1.65 | 0.67 ± 0.17 |
| Hypopnea index (counts/hour) | 5.01 ± 1.78 | 0.95 ± 0.41 |

As will be apparent from these results, neither sleeping time nor sleep efficacy was changed significantly. In contrast, both the absolute counts of apnea and hypopnea as well as the apnea index and the hypopnea index decreased significantly.

In view of the fact that the original aim of the study was to investigate the efficacy of the extract combination, further studies were made to investigated the contributions of each of the extracts of the combination. This was done by means of in vitro studies reported below and supported the teaching that beneficial results as regards sleep-induced apnea and hypopnea will be obtained when Humulus lupulus extract is omitted, but not vice versa indicating that the constituents of the valerian extract were essential for the invention.

### Example 2

A series of experiments was made to determine the binding of candidate substances to adenosine A₁ receptors. The method used was essentially as reported in Biochemical Studies of CNS Receptors (Handbook of Psychopharmacology, Vol. 17) Plenum Press, New York 1983.

The following substances were used in the tests:
a) dry extract of Valeriana officinalis, Zeller Batch No. 011540
b) dry extract of Valeriana officinalis, compacted, Zeller Batch No.012270
c) dry extract of Humulus lupulus, Zeller Batch No.12450
d) mixture of dry extracts a) and c) (weight ratio 250:60)
e) extract solution 0.01 g/ml - Test VOB 58
f) extract solution 0.01 g/ml - Test VOB 79
g) Valerian constituent "Scholle-4", a lignan-type olivil derivative isolated from Valeriana officinalis.

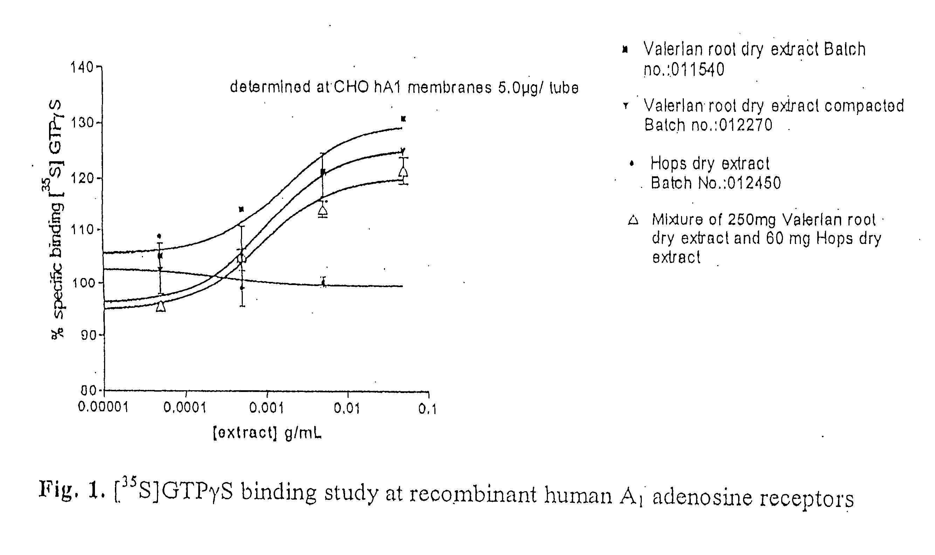

For comparative purposes N⁶-cyclopentyladenosine (CPA), a full A₁ antagonist, was used because of its known characteristics in the tests.
(A) Stimulation of [³⁵S]GTPγS binding to recombinant human A₁ adenosine receptors expressed in Chinese hamster ovary (CHO) cells was tested on substances a), b), c), and d). Kᵢ and IC₅₀ values from binding experiments on rat A₁ adenosine receptors are included for comparison. The results are given in Table 2 below and in Fig.1. As will become apparent from these results, the binding effectivity of extracts of Humulus lupulus is not detectable per se, and it is only the valerian extract that exhibits distinctive binding. However, some synergy of the combined extracts is believed to be supported by these tests and it can be assumed that other combinations of valerian extract with plant extracts that have an activity comparable to that of Humulus lupulus will show a similar performance. Examples of such other plants are Melissa, Passiflora, and Petasites.

**Table 2**

| Stimulation of [³⁵S]GTPγS binding to recombinant human A₁ adenosine receptors expressed in Chinese hamster ovary (CHO) cells (Kᵢ and IC₅₀ values from binding experiments at rat A₁ adenosine receptors are included for comparison) | | | |
|---|---|---|---|
| A₁ Adenosine receptor affinity rat cortical brain membranes vs. [³H]CCPA | | | Stimulation of [³⁵S]GTPγS binding at CHO-hA₁ adenosine receptors |
| | Kᵢ ± SEM [mg/mL] n=2 | IC₅₀ ± SEM [mg/mL] n=2 | IC₅₀ ± SEM [mg/mL] n=2 |
| a) Valerian extract not compacted | 0.15 ± 0.001 | 0.53 ± 0.01 | 0.85 ± 0.05 |
| b) Valerian extract compacted | 0.19 ± 0.08 | 0.66 ± 0.24 | 1.2 ± 0.8 |
| c) Humulus lupulus | 0.13 ± 0.07 | 0.40 ± 0.25 | No stimulation |
| c) Valeriana/Humulus lupulus (mixture) | 0.37 ± 0.16* | 1.32 ± 0.59* | 0.86 ± 0.33 |

| | | | |
|---|---|---|---|
| Note: *n=3 | | | |

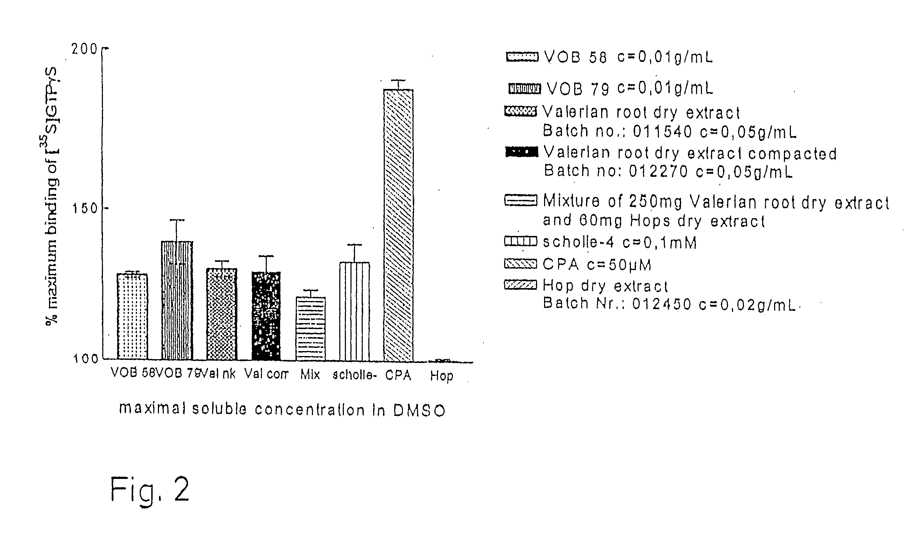

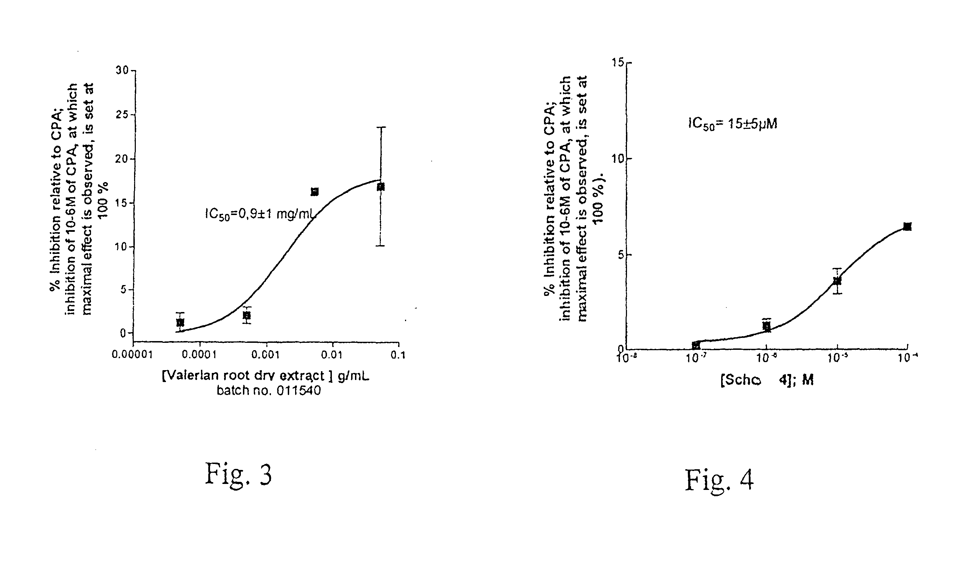

(B) In another sequence of tests, the test substances mentioned above were tested for maximum effects of [³⁵S]GTPγS stimulation on human recombinant A₁ adenosine receptors (hA,-CHO cell membranes). All test materials were tested in the concentration that produced maximum effects. The results of the lignan-type olivil derivative obtained from valerian extract termed "scholle-4" are shown in Fig. 2 as well.
(C) Adenylacetylase assays were made in the manner specified in the literature cited above to determine inhibition of the forskoline-stimulated cAMP formation by A₁ adenosine receptor antagonists, measured on CHO cell membranes that express the human A₁ adenosine receptor. The results are shown in Fig. 3 for the dry valerian extract and in Fig. 4 for the olivine derivative (scholle-4).

It is to be emphasized that the preceding examples and other exemplifications were given for purposes of illustration and not for limitation and the scope of the invention is to be construed on the basis of the following claims.

## Claims

1. A use of an extract from Valeriana officinalis for preparation of a pharmaceutical composition for treatment of sleep-induced apnea or hypopnea in a human patient.

2. The use of claim 1 wherein said extract from Valeriana officinalis is combined with an extract of at least one plant selected from the group including Humulus, Melissa, Passiflora, and Petasites.

## Patentansprüche

1. Verwendung eines Extraktes aus Valeriana officinalis für die Herstellung einer pharmazeutischen Zubereitung für die Behandlung von Schlafapnoe oder Hypopnoe an einem Humanpatienten.

2. Verwendung gemäss Anspruch 1, **dadurch gekennzeichnet, dass** der Extrakt aus Valeriana officinalis mit einem Extrakt aus mindestens einer Pflanze der Gruppe Humulus, Melisse, Passiflora und Petasites kombiniert wird.

## Revendications

1. Utilisation d'un extrait de *Valeriana officinalis* destiné à la préparation d'une composition pharmaceutique en vue du traitement de l'apnée ou l'hypopnée déclenchée par le sommeil chez un patient humain.

2. Utilisation de la revendication 1, dans laquelle ledit extrait de *Valeriana officinalis* est combiné avec un extrait d'au moins une plante choisie dans le groupe comprenant l'humulus, la mélisse, la passiflore et la pétasite.
